Europäisches Patentamt

European Patent Office

Office européen des brevets

(11) Veröffentlichungsnummer: **0 473 011 A2**

# EUROPÄISCHE PATENTANMELDUNG

(21) Anmeldenummer: **91113641.4**

(22) Anmeldetag: **14.08.91**

(51) Int. Cl.5: **C12N 9/00**, C12M 1/04

(30) Priorität: **29.08.90 DE 4027290**

(43) Veröffentlichungstag der Anmeldung:
**04.03.92 Patentblatt  92/10**

(84) Benannte Vertragsstaaten:
**AT CH DE DK LI NL**

(71) Anmelder: **Forschungszentrum Jülich GmbH**
**Postfach 1913, Wilhelm-Johnen-Strasse**
**W-5170 Jülich(DE)**

(72) Erfinder: **Kula, Maria-Regina, Prof.**
**Selgenbusch 12**
**W-5162 Niederzier/Hambach(DE)**
Erfinder: **Grote, Martin Roger**
**Hedwigstrasse 1**
**W-4670 Lünen(DE)**

(54) **Verfahren und Vorrichtung zur Gewinnung von Enzymen aus enzymhaltigen Suspensionen.**

(57) Zur Gewinnung von Enzymen aus einer enzymhaltigen Suspension, wie insbesondere aus unbehandelter Fermentationsbrühe, gibt man die Suspension zusammen mit enzymanlagerungsfähigem Kornmaterial, wie insbesondere einem Ionenaustauschermaterial, in ein senkrechtes Rohr und leitet durch die Masse einen insbesondere durch trockenen Stickstoff gebildeten Inertgasstrom mit einer zur Aufwirbelung ausreichenden Gasgeschwindigkeit. Dabei wird die Masse durchmischt und Lösungsmittel (Wasser) verdunstet bis eine ausreichende Enzymanlagerung am Kornmaterial durch Aufkonzentrierung erreicht ist. Die Suspensionsreste werden insbesondere durch einen Druckgasstoß aus der Masse ausgetrieben und das angelagerte Enzym eluiert. Zur Beschleunigung des Verfahrens werden vorzugsweise Gasstrom und/oder die Masse selbst auf enzymverträgliche Temperaturen aufgeheizt. Vorzugsweise kann zwischen der Entfernung der Suspensionsreste und der Elution des Enzyms ein Waschvorgang erfolgen, wobei die Waschflüssikeit zweckmäßigerweise über eine weitere Festbettsäule mit enzymanlagerungsfähigem Kornmaterial gegeben wird. Die Elution kann als Stufenelution durchgeführt werden.

FIG. 1

Die Erfindung bezieht sich auf ein Verfahren zur Gewinnung von Enzymen aus einer enzymhaltigen Suspension, insbesondere aus unbehandelter Fermentationsbrühe.

Für die Gewinnung von Enzymen aus Fermentationsbrühen sind unterschiedliche Verfahrensweisen bekannt. Ganz allgemein wird dabei üblicherweise zunächst Biomasse durch Zentrifugieren oder Filtrieren abgetrennt und die erhaltene Flüssigkeit durch Ultrafiltration konzentriert (E. Flaschel et al, Advances in Biochem. Engng. Biotechn. 26 (1983) 73-142).

Bei dieser bekannten Arbeitsweise geht bei der anfänglichen Separation von Biomasse ein nicht unerheblicher Anteil des gebildeten Enzyms verloren, und die beiden Separationsschritte erfordern Zeit und Aufwand.

Ziel der Erfindung ist daher eine Enzymgewinnung, bei der Enzymverluste möglichst gering gehalten werden können, bei möglichst geringem technischen Aufwand.

Das zu diesem Zweck entwickelte erfindungsgemäße Verfahren ist dadurch gekennzeichnet, daß man die Suspension mit enzymanlagerungsfähigem Kornmaterial in einem senkrechten Behälter, insbesondere Rohr, zusammenbringt und durch die Masse einen aufsteigenden Inertgasstrom mit einer zur Aufwirbelung ausreichenden Gasgeschwindigkeit unter Durchmischung und Verdunstung hindurchleitet bis zur ausreichenden Anlagerung des Enzyms am Kornmaterial, daß man dann die Suspensionsreste aus der Masse entfernt und des angelagerte Enzym eluiert.

Bei diesem Verfahren wird die Biomasse nicht vorher abgetrennt, sondern verbleibt in der zu verwerfenden Restflüssigkeit, aus der das Enzym selektiv durch Anlagerung "entnommen" worden ist. Die Anlagerung der Enzyme erfolgt an einem dazu befähigten Kornmateriel, wie biospezifischen Adsorbentien für die Affinitätschromatographie oder an einem hydrophoben Träger und insbesondere an geeigneten Ionenaustauschermaterialien, wodurch für eine weitgehende Enzymverarmung der biomassehaltigen Restsuspension gesorgt wird, die dann vom enzym-angereicherten Kornmaterial getrennt werden kann, z.B. durch Beschleunigung mittels Druckgradienten.

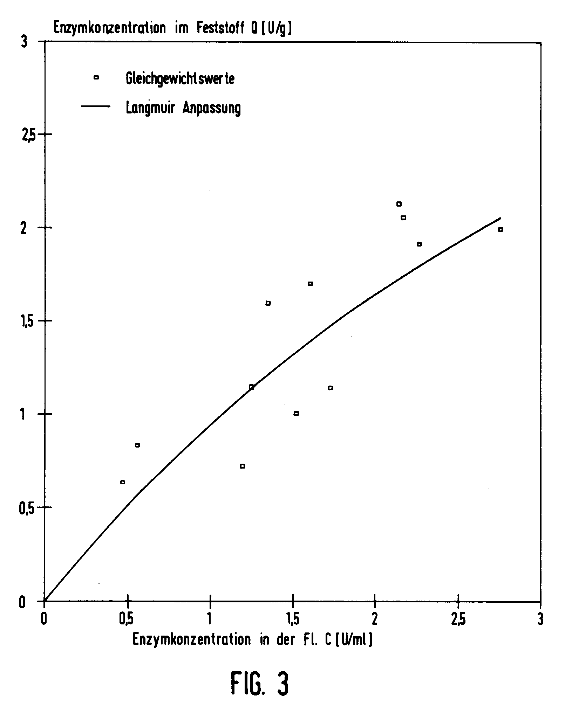

Um eine ausreichende Durchmischung und gleichzeitig eine Aufkonzentrierung der gelösten bzw. anzulagernden Enzyme zu erreichen, wird die Suspension/Kornmaterial-Mischung von einem aufsteigenden trockenen Inertgasstrom durchwirbelt (bzw. "aufgeschäumt"), bis eine Aufkonzentrierung des Enzyms durch Verdunstung und dadurch eine ausreichende Anreicherung des Enzyms am Kornmaterial erfolgt ist. Dieser Schritt wird insbesondere unter Beachtung der Enzymstabilität ggf. unter Wärmezufuhr derart durchgeführt, daß keine wesentlichen Aktivitätsverluste durch Desaktivierung auftreten, sowie unter Beachtung der (konzentrationsabhängigen) Anlagerungsgleichgewichte (siehe Fig. 3) zwischen gelöstem Enzym und angelagertem Enzym.

Die für die Enzymanlagerung notwendige Granulatmenge richtet nach dem zu verarbeitenden Suspensionsvolumen, der Anlagerungsaffinität und -kapazität des Kornmaterials sowie der Stabilität des Enzyms.

Die Korngröße ist nicht kritisch. Zur Zeit werden Granulate von 40 bis 150 $\mu$m Korngröße aus Gründen der Verfügbarkeit benutzt. Mit zunehmender Korngröße sinkt allerdings naturgemäß die Anlagerungskapazität, was ggf. zu berücksichtigen ist. Poröse Materialien wie z.B. anlagerungsspezifisch beschichtete offenporige Sinterglasperlen sind nützlich. Auch die Kornform ist frei wählbar.

Grundsätzlich wird aus Wirtschaftlichkeitsgründen eine möglichst geringe Granulatmenge vorgesehen, deren Anlagerungskapazität jedoch für die (bis zum Elutionsschritt) aus der vorhandenen Suspension aufzunehmende Enzymmenge ausreichen soll.

Ist die Anlagerungsaffinität des Granulats gering und damit die im Suspensionsrest verbleibende Enzymkonzentration hoch, sollte die Eindunstung möglichst weit getrieben werden, bevor der Suspensionsrest aus der Säule entfernt wird. Experimentell konnten Mengenverhältnisse von Flüssigkeitsrest zu Granulat bis herab zu 0,08 ml/g erreicht bzw. verarbeitet werden.

Bei der Zugabe der Suspension zum equilibrierten Granulat wird zweckmäßigerweise ein Mindestverhältnis von Suspension zu Granulat (von z.B. 0,3 ml/g) angestrebt, das mit einer brauchbaren Durchmischung beider Komponenten mit Hilfe des Gasstroms vereinbar ist. (Anderenfalls muß zu Beginn mechanisch gerührt werden.) Sehr viel höhere Verhältnisse von zugegebener Suspensionsmenge zum Granulat in der Säule erscheinen nicht angemessen; vielmehr wird dann zweckmäßigerweise mit portionsweiser Zugabe und Zwischenverdunstung gearbeitet, um die Säulenhöhe zu begrenzen. Die portionsweise Zugabe bietet zudem Vorteile bei relativ geringer Enzymstabilität, weil die globale Verweilzeit der Gesamtmenge des am Granulat anzureichernden Enzyms auf diese Weise minimiert werden kann. Dies spielt insbesondere eine Rolle, wenn unter Temperaturerhöhung verdunstet wird.

Für den Gasstrom eignet sich Inertgas, wie z.B. Stickstoff, insbesondere in getrockneter Form,um die Verdunstung zu beschleunigen. Eine möglichst reichliche Gaszufuhr und gute Gasverteilung sind nützlich für die gewünschte gute Durchmischung und rasche Verdunstung. Die Blasengröße hängt u.a. von der Voskosität der behandelten Mischung ab. Blasengrößen im Bereich von ca. 0,2 bis 1 cm sind zweckmäßig.

Die Abtrennung der biomassehaltigen Suspensionsreste vom Kornmaterial erfolgt insbesondere durch Auspressen mittels Druckgas.

Das so von Biomasse und anhängenden Flüssigkeitsresten getrennte Kornmaterial wird ggf. gewaschen und dann mit einem Elutionsmittel zur Enzymelution behandelt, wobei insbesondere durch Stufenelution für eine fraktionierte Elution unterschiedlicher Enzyme gesorgt wird.

Weitere Besonderheiten der Erfindung gehen aus den Patentansprüchen sowie der nachfolgenden Beschreibung von Ausführugnsbeispielen hervor, wobei auf die angefügten Zeichnungen Bezug genommen wird. Es zeigen:

| | |
|---|---|
| Figur 1 | eine Labor-Anordnung zur Durchführung des erfindungsgemäßen Verfahrens; |
| Figur 2 | Schemata zur Veranschaulichung der einzelnen Phasen des erfindugnsgemäßen Verfahrens; |
| Figur 3 | Gleichgewichtswerte zwischen adsorbiertem und in der Suspension befindlichem Enzym; |
| Figur 4 u. 5 | Schaubilder für die Enzymmengenbilanz bei zwei unterschiedlichen Verfahrensweisen; |
| Figur 6 u. 7 | Protease-Elutionskurven |
| Figur 8 | Absorptionsspektren für eluiertes Enzym im Vergleich zur Fermentationsbrühe und käuflicher Protease; |
| Figur 9 | eine Kurvenschar für die Befüllung der Enzymanlagerungssäule für das System Protease/Fractogel; |
| Figur 10 | ein Diagramm für die angelagerte Enzymmenge in Abhängigkeit von der Aufkonzentrierung der Flüssigkeit durch Verdunstung; und |
| Figur 11 | die Enzym-Aufreinigung in Abhängigkeit von der prozentualen Verdunstung. |

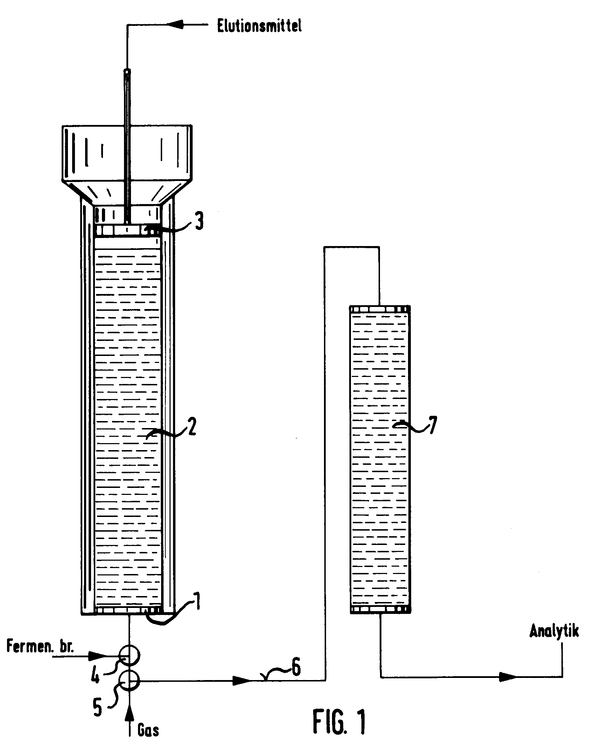

Im Labormaßstab kann das erfindungsgemäße Verfahren insbesondere in einer Experimentieranordnung durchgeführt werden, wie sie durch Figur 1 veranschaulicht wird. Dabei wird eine mit Siebboden 1 versehene heizbare Säule 2 durch einen beweglichen Stempel 3 abgeschlossen, der für eine gleichverteilte Elutionsmittelzufuhr geeignet ist. Unterhalb der Säule 2 kann bei 4 Suspension, insbesondere Fermentationsbrühe, injiziert werden, und über 5 wird Gas in die Säule geschickt. Die zu verarbeitende Suspension könnte selbstverständlich auch an anderer Stelle eingespeist werden. Über eine Verbindungsleitung 6 kann der Ablauf der Säule 2 auf eine anschließende Festbettsäule 7 gegeben werden, die für eine fraktionierte Elution geeignet ist. Am oberen Ende hat die Säule 2 vorzugsweise eine Erweiterung, insb. konische Erweiterung, zur Verlangsamung des Gasstroms, wodurch ein Mitreißen von Füllung durch den austretenden Gasstrom vermieden wird. Das untere Ende kann ggf. konisch verjüngt sein.

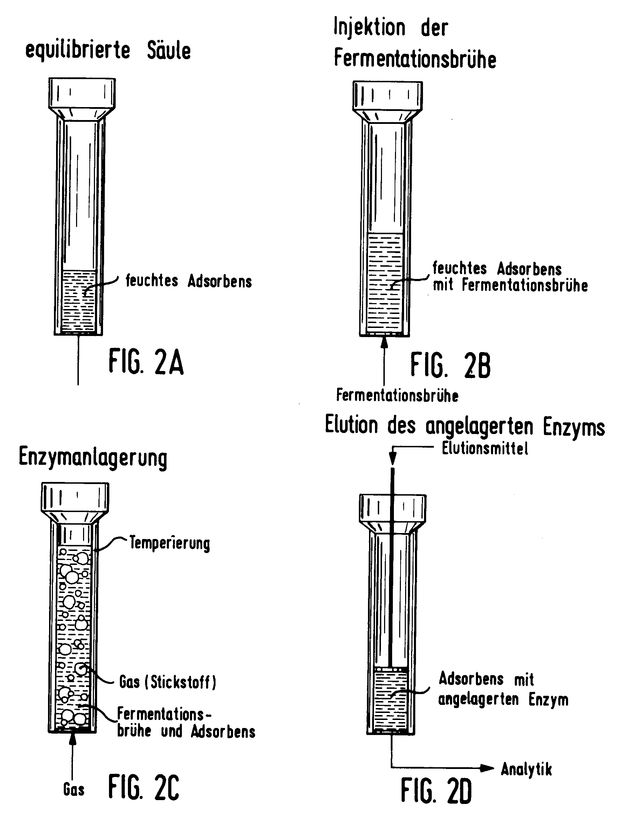

Figur 2 veranschaulicht die wesentlichen Phasen der Enzymgewinnung: Gemäß (a) ist die Säule 2 mit feuchtem equilibrierten Kornmaterial versehen, in das insbesondere durch Injektion von unten her Fermentationsbrühe eingeführt wird (b). Die Mischung von Fermentationsbrühe und Kornmaterial wird dann durch Gas aufgewirbelt bzw. "aufgeschäumt" (c), ggf. unter Wärmezufuhr über erwärmtes Gas oder einen entsprechenden Heizmantel, wobei eine gute Durchmischung und Verdunstung von Lösungsmittel (Wasser) erreicht wird. Wärmezufuhr und Dauer richten sich in dieser Verfahrensstufe nach der Stabilität des Enzyms bzw. der Enzyme einerseits und der anzustrebenden Aufkonzentrierung der Flüssigkeit zur Erzielung einer möglichst weitgehenden Enzymanlagerung am Kornmaterial. Anschließend an die Phase (c), die bei ausreichender Enzymstabilität und nur mäßiger Affinität des Kornmaterials zum Enzym (die dann eine möglichst weitgehende Enzym-Aufkonzentrierung der Flüssigkeit nötig macht) soweit getrieben wird, bis eine quasi-Viskose Masse resultiert, wird die biomassehaltige Restsuspension mittels eines durch Umleiten des Gases zum Säulenkopf realisierbaren Druckaufbaus unter Druckentlastung aus der Säule herausgepreßt.

Danach wird das feuchte Kornmaterial vorzugsweise kurz gewaschen und dann, wie in (d) veranschaulicht ist, mit Elutionsmittel zur ggf. fraktionierten Elution des Enzyms bzw. der Enzyme beaufschlagt.

Die Waschflüssigkeit wird zweckmäßigerweise nicht verworfen, sondern unmittelbar auf die anschließende Festbettsäule 7 (Figur 1) gegeben, so daß in der Waschflüssigkeit enthaltene Enzymanteile für die Enzymgewinnung nicht verlorengehen, die dann insgesamt durch Zufuhr von Elutionsmittel erfolgt. Die Säule 7 enthält ebenfalls enzymanlagerungsfähiges Kornmaterial.

Beispiel 1

Verarbeitet wurde Fermentationsbrühe von pH 7 mit Bacillus licheniformis und gebildeter extrazellulärer alkalischer Protease folgender Zusammensetzung des "synthetischen Mediums":

| Glycerin (87%) | | 22,971 g |
| Amm niumsulfat | | 6,000 g |
| Kaliumdihydrogenphosphat | | 2,721 g |
| Kaliumhydroxid | | 0,561 g |
| Calciumchlorid | | 0,073 g |
| Magnesiumsulfat • 7 $H_2O$ | | 0,246 g |
| Eisensulfat • 7 $H_2O$ | | 0,027 g |
| Mangansulfat • $H_2O$ | | 0,008 g |
| Spurenelemente | | 0,010 l |
| $H_2O$ bidest | ad | 1,000 l |
| Nährstoffe | $\Sigma =$ | 26,000 g |

In einer Apparatur gemäß Figur 1 mit einer Höhe der Säule 2 von 300 mm und einem Innendurchmesser von 26 mm wurden 50 g Ionenaustauscherharz (Fractogel EMD $SO_3$ -650 (C), Fa. Merck, Darmstadt) mit einer Korngröße im Bereich von 90 - 150 $\mu$m mit 200 ml Puffer ($KH_2PO_4$ - $Na_2HPO_4$ Puffer; pH 5; 67 mM) equilibriert. Nach der Equilibrierung wurde der Puffer aus der Säule abgelassen und Fermentationsbrühe

mit einer Spritze von unten injiziert. Danach wurde von unten ein regelbarer Gasstrom (Stickstoff) eingeleitet, der den Ionenaustauscher mit der Fermentationsbrühe unter Aufwirbelung durchmischt. Durch Erwärmung der Säule auf bis zu 50 °C wurde für eine Verdunstung der Anlagerung gesorgt. Nach etwa 15 - 60 Min. wurde die restliche Fermentationsbrühe aus der "Wirbelschichtadsorptionskolonne" entfernt.

Am vollständigsten wird dieses durch ein Umleiten des Gasstromes in den vorher abgedichteten oberen Teil der Kolonne erreicht, wodurch ein Druck von 1 bar im Kolonnenkopf aufgebaut wird. Nach dem Öffnen des unteren Ablaufes wurde hierdurch die Fermentationsbrühe mit großer Geschwindigkeit aus dem Adsorber gepreßt und verworfen, wobei der größte Teil der nicht angelagerten Biomasse vom Kornmaterial entfernt wurde. Das feuchte Fractogel wurde mit 100 ml Wasser gewaschen. Diese Waschflüssigkeit kann ggf. verworfen werden, sie wurde jedoch vorzugsweise über die zusätzliche Festbettkolonne 7 geleitet (was wegen des geringen Biomassenanteils möglich ist, ohne daß eine Verstopfung der Säule zu befürchten wäre), in der die bei dem Waschvorgang desorbierten Enzyme wieder "adsorbiert" bzw. festgehalten werden

Für die Enzymelution wurde die Säule 2 in eine Festbettkolonne umgerüstet (Figur 2d): Dieses erfolgte durch Herablassen eines Stempels, der bis zur Schüttungshöhe des Kornmaterials geführt wurde. Hierbei ist darauf zu achten, daß zwischen den Partikeln genügend Waschwasser verbleibt, um Luftblasen zu vermeiden. Die Elutionsflüssigkeit wurde dann von oben in die Säule 2 geleitet und der Ablauf durch die zusätzliche Festbettkolonne 7 geführt. Mit einem Fraktionssammler wurde das eluierte Enzym (Desorbat) zur weiteren Analyse gesammelt.

In der beschriebenen Weise und mit der beschriebenen Anordnung wurden unter folgenden Versuchsbedingungen unterschiedliche Versuche durchgeführt:
Es wurden Fermentationsbrühen zur Proteasegewinnung aus unterschiedlichen Fermentationen verwendet (A u. B). Das Fractogel wurde vor jedem Versuch mit dem Puffer $KH_2PO_4$ - $Na_2HPO_4$ auf pH 5 equilibriert. Bei allen Versuchen wurden 15 ml Fermentationsbrühe eingesetzt. Die Fermentationsbrühen wurden mit 50 g Fractogel vermischt. Als Waschflüssigkeit wurde destilliertes Wasser verwendet.

Die Elution erfolgte entweder durch eine kontinuierliche (mit "Gradient") oder durch eine stufenweise Erhöhung der Salzkonzentration und Erhöhung des pH-wertes. Nach den Versuchen wurde das Fractogel mit 0,5 m Natronlauge regeneriert. Beim Versuch 1 wurde mit dem Puffer $Na_2HPO_4$-NaOH pH 11 mit 1 m NaCl eluiert. Unterschiedliche Salzkonzentrationen wurden bei den Versuchen 2 bis 5 als Elutionsmittel gewählt. Bei dem Versuch 5 wurde die Waschflüssigkeit durch die zusätzliche Festbettkolonnne geleitet, die mit 50 g Fractogel gefüllt war.

In der nachfolgenden Tabelle 1 sind einzelne Parameter der Versuche sowie die Protease-Konzentrationen der eingesetzten Fermentationsbrühen aufgeführt.

Tabelle 1

| Versuch | Protease-Konzentration | Art der Desorption | Säulen-Temp. | Verdunstung | Fementation |
|---------|------------------------|--------------------|--------------|-------------|-------------|
| 1 | 1571 PE/ml* | Gradient | 20 °C | 43,3 % | A |
| 2 | 2554 PE/ml | Stufen | 40 °C | 56,7 % | B |
| 3 | 2524 PE/ml | Stufen | 50 °C | 77,3 % | B |
| 4 | 2282 PE/ml | Stufen | 40 °C | 44,0 % | B |
| 5 | 2589 PE/ml | Stufen | 50 °C | 72,0 % | B |

* 348 PE/ml = 1 U/ml (Unit nach Anson, J. Gen. Physiol. 22 (1938) 79-89)

In der nachfolgenden Tabelle 2 sind die adsorbierten und desorbierten Enzymmengen bezogen auf die vor der Adsorption in der Fermentationsbrühe vorhandene Enzymmenge angegeben.

Tabelle 2

| Versuch | adsorbierte Enzymmenge | desorbierte Enzymmenge |
|---------|------------------------|------------------------|
| 1 | 84,5 % | --- *) |
| 2 | 87 % | 55,9 % |
| 3 | 91,4 % | 56,6 % |
| 4 | 86,1 % | 56,4 % |
| 5 | 91,1 % | 78,7 % |

*) Enzym bereits beim Waschvorgang völlig desorbiert.

Unter den Versuchsbedingungen (15 ml Fermentationsbrühe, 50 g Fractogel) konnte wegen der Erhöhung der Viskosität maximal ca. 75 % der eingesetzten Fermentationsbrühe verdunstet werden. Die dabei maximal erreichbare angelagerte Enzymmenge lag zwischen 91 und 92 %. Die Erhöhung der Adsorption, die durch Verdunstung erreicht wurde, ist in Fig.3 aufgetragen.

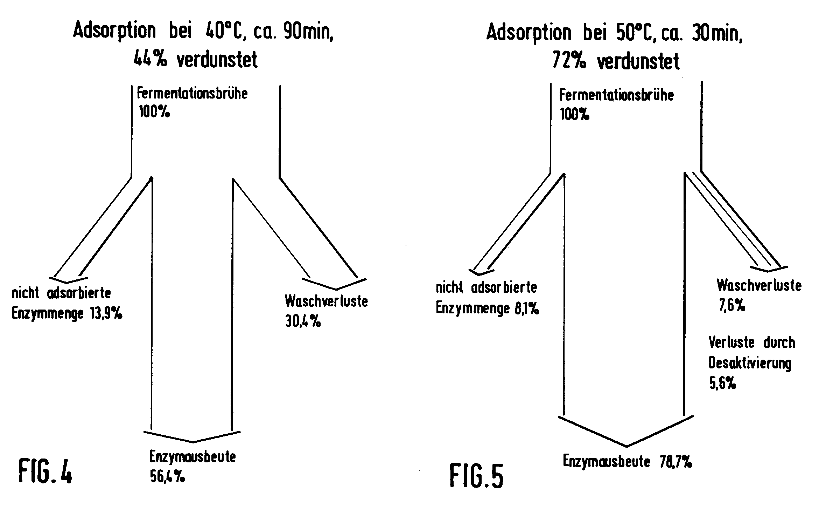

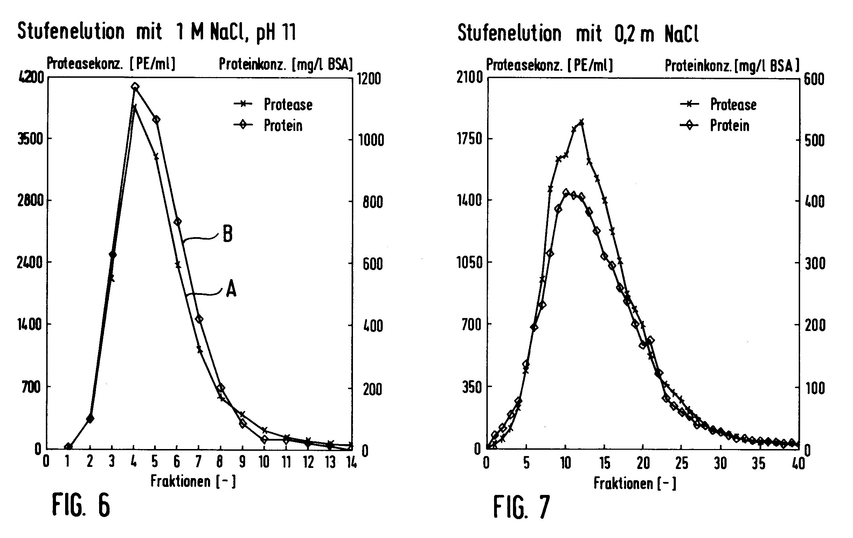

Mit der zusätzlichen Festbettkolonne können die Waschverluste reduziert werden. Dies wird durch Vergleich der Enzymmengenbilanz Von Versuch 4 (Fig. 4) mit derjenigen vom mit Zusatzkolonne durchgeführten Versuch 5 (Figur 5) verdeutlicht: Die im Desorbat befindliche Proteasemenge steigert sich bezogen auf die ursprüngliche Proteasemenge in der Fermentationsbrühe von 56,4 % auf 78,7 %, siehe nahfolgende Tabelle 3.

Tabelle 3

| Versuch | eingesetzte Fermentationsb. | | nicht adsorb. Enzymmenge | | Waschverluste | | Hitze-Desak- tivierung | | desorbierte Enzymmenge | |
|---------|-----|-----|-----|-----|-----|-----|-----|-----|-----|-----|
| | U | % | U | % | U | % | U | % | U | % |
| 4 | 94,4 | 100 | 13,7 | 13,9 | 29,9 | 30,4 | | | 55,5 | 56,4 |
| 5 | 111,6 | 100 | 9,0 | 8,1 | 8,5 | 7,6 | 6,3 | 5,6 | 87,8 | 78,7 |

| Versuch | Adsoptions- temperatur | Verdunstung | Adsoptionsdauer | zusätzliche Adsorbtionskolonne für die Waschflüssigkeit |
|---------|----------|-------------|-----------------|----------------------------------------------|
| | °C | % | s | |
| 4 | 40 | 44 | 5400 | nicht eingesetzt |
| 5 | 50 | 72 | 1800 | eingesetzt mit 50g Adsorbens (feucht) |

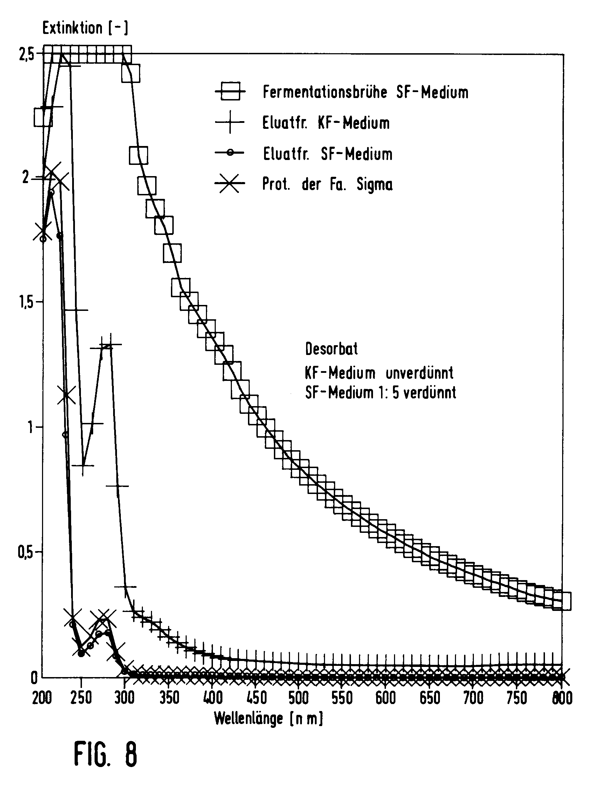

Die Figuren 6 und 7 veranschaulichen den Erfolg des erfindungsgemäßen Verfahrens: Dabei zeigt die Figur 6 das Elutionsprofil einer von Bacillus lichniformis herstammenden Protease bei Nachweis der Proteaseaktivität (A) und des Proteingehalts im Eluat (B). Beide Kurven A und B zeigen einen gleichen Verlauf.

Figur 8 zeigt Absorptionsspektren für die Fermentationsbrühe, das als Eluat gewonnene Produkt und für käufliche Protease der Firma Sigma: Wie man sieht, gleicht das Absorptionsspektrum des erfindungsgemäßen Produkts demjenigen der Protease von Sigma, während die Fermentationsbrühe ein völlig anderes Absorptionsspektrum ergibt. Der gleichartige Verlauf des Absorptionsspektrums des Eluats und der Protease von Sigma zeigt, daß der Anteil an Fremdprotein im Eluat außerordentlich gering ist.

Dieses Ergebnis konnte durch die Durchführung einer Gel-Elektrophorese bestätigt werden. Das Eluat weist ebenso wie die Protease eine Bande bei ≈ 27500 Dalton auf. Verunreinigungen sind bei beiden Proben nicht zu erkennen.

Die spezifische Aktivität des Produktes war sogar bis zum Anderthalbfachen höher als bei der käuflichen Protease der Fa. Sigma.

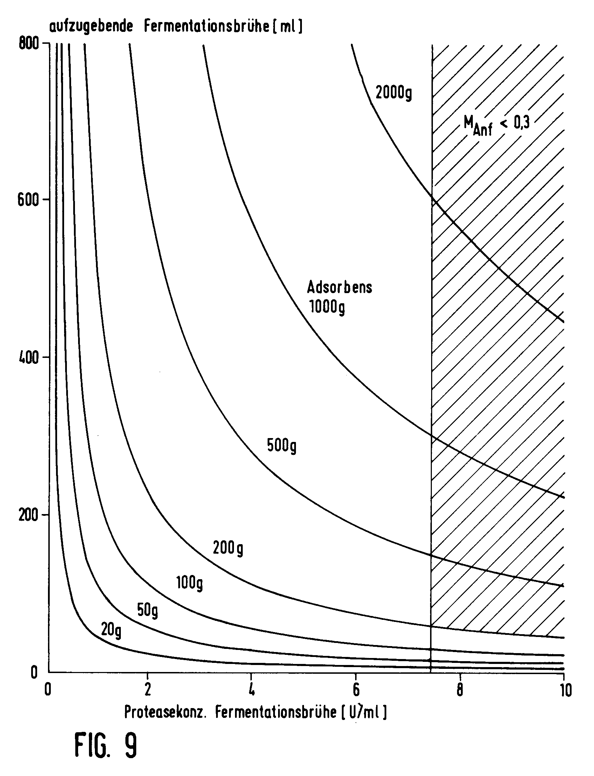

Die Auslegung einer erfindungsgemäß zu betreibenden Kolonne zur Granulat-Fixierung von Enzymen aus biomassehaltigen Suspensionen im Hinblick auf optimale Wirtschaftlichkeit (maximale Ausbeute an Enzym und maximale Enzymbelegung des Granulats) wurde für das Beispiel Protease/Fractogel der Fa. Merck errechnet (siehe Fig. 9): Dabei wurde als maximale Proteaseanlagerung der bislang erreichte (noch zu verbessernde) Wert von 2 U/g bei einer Proteasekonzentration in der Restflüssigkeit von 2,6 U/ml zugrundegelegt.

Das (noch brauchbare) Mengenverhältnis von Fermentationsbrühe zu Granulat bei der Eingabe der Suspension wurde zu 0,3 ml/g angesetzt, da bei geringeren Werten die anfängliche Durchmischung von feuchtem Granulat und injizierter Flüssikeit schwieriger wird. Nach der erfindungsgemäßen Aufkonzentrierung des Enzyms in der Flüssigkeit sollte das Verhältnis von Restflüssigkeit zu Granulat möglichst niedrig sein, um Enzymverluste durch die Abtrennung der Restflüssigkeit möglichst gering zu halten. In praxi konnten minimale Verhältnisse von 0,08 ml/g erreicht werden. Diese beiden Verhältnisse gehen ebenfalls in die durchgeführte Berechnung ein.

Die dargestellte Kurvenschar zeigt nun den jeweils notwendigen Granulatbedarf in Abhängigkeit vom Volumen der Fermentationsbrühe und ihrer Proteasekonzentration für eine maximale Ausbeute an Enzym und eine maximale Beladung des Granulats. Die schraffierte Fläche gibt den Bereich an, an dem des anfängliche Mengenverhältnis kleiner als 0,3 ist. Bei diesem Mengenverhältnis ist eine anfängliche Durchmischung von Adsorbens und der in den Adsorber geleitete Fermentationsbrühe nur mit zusätzlichem Rühren möglich.

Bei dieser erfindungsgemäßen Durchführung sollte das Fassungsvermögen der Säule (2) mindestens doppelt so groß sein wie der vom Granulat einzunehmende Raum, da die Zunahme der Füllung durch die (vorzugsweise von unten) zugegebene Suspension und durch die Expansion infolge des eingeblasenen Gases bei mindestens 100 % liegt. Größere Säulenhöhen können ohne weiteres angewandt werden. Die Suspensionsmenge, die zur Erreichung der berechneten Betriebspunkte notwendig ist, kann auf einmal zugegeben werden oder protionsweise mit Zwischenverdunstung, wenn das Fassungsvermögen der Säule bei Durchwirbelung mit Gas für die gesamte aufzugebende Flüssigkeitsmenge nicht ausreicht.

Beispiel 2

Analog zu Beispiel 1 wurde Fermentationsbrühe mit komplexem Medium von pH 7 folgender Zusammensetzung aufgearbeitet (KF-Medium):

| | |
|---|---|
| Maisstärke | 90,000 g |
| $\alpha$–Amylase | 0,100 g |
| Natrium–Kaseinat | 25,000 g |
| Sojamehl | 19,000 g |
| Maisquellwasser | 7,000 g |
| Ammoniumdihydrogenphosphat | 0,500 g |
| di–Natriumhydrogenphosphat | 0,500 g |
| Kaliumhydrogenphosphat | 0,200 g |
| Eisensulfat • 7 $H_2O$ | 0,048 g |
| Mangansulfat • $H_2O$ | 0,020 g |
| $H_2O$ | ad 1,000 l |
| Nährstoffe | $\Sigma =$ 141,000 g |

In diesem Medium wird neben der alkalischen Protease auch eine $\alpha$-Amylase vom Bacillus licheniformis extrazellulär gebildet. Die Trennung der alkalischen Protease aus dem komplexen Fermentationsmedium ist durch den wesentlich höheren Nährstoffanteil (Faktor 5) und durch die Anwesenheit eines zweiten extrazellulären Enzyms gegenüber der Aufarbeitung des synthetischen Fermentationsmediums (SF-Medium) gemäß Beispiel 1 erschwert.

In den nachfolgenden Tabellen 4 und 5 sind verschiedene Parameter der durchgeführten Versuche zusammengestellt.

Tabelle 4

| Versuch | $C_{FB}$ | $C'_{FB}$ | desorbierte Enzymmenge | Ausbeute | aufgegebene Fermentations—brühe |
|---------|----------|-----------|------------------------|----------|--------------------------------|
|         | [PE/l]   | [PE/l]    | [PE]                   | [%]      | [ml]                           |
| 1  | 2631 | 1540 | 21030 | 39,3 | 20  |
| 2  | 2099 | 980  | 19341 | 61,4 | 15  |
| 3  | 2329 | 998  | 21255 | 60,3 | 15  |
| 4  | 2146 | 1036 | 22277 | 69,0 | 15  |
| 5  | 2578 | 1206 | 20840 | 53,9 | 15  |
| 6  | 2547 | 1499 | 25160 | 49,4 | 20  |
| 7  | 2004 | 1063 | 18569 | 61,8 | 15  |
| 8  | 2281 | 699  | 26891 | 78,6 | 15  |
| 9  | 2486 | 2180 | 22357 | 36,0 | 25  |
| 10 | 2354 | 1833 | 21286 | 45,2 | 20  |
| 11 | 1914 | 1967 | 19687 | 34,3 | 30  |
| 12 | 2826 | 2044 | 22838 | 27,1 | 30  |
| 13 | 1940 | 897  | 19827 | 68,2 | 15  |
| 14 | 2609 | 978  | 21117 | 54,0 | 15  |
| 15 | 1956 | 655  | 15791 | 53.8 | 15  |
| 16 | 2335 | 686  | 19583 | 55,9 | 15  |
| 17 | 2587 | 734  | 22858 | 58,9 | 15  |
| 18 | 2638 | 2607 | 28350 | 30,7 | 35  |
| 19 | 2947 | 565  | 15360 | 69,5 | 7,5 |
| 20 | 2524 | 350  | 13482 | 71,2 | 7,5 |
| 21 | 2631 | 2519 | 30476 | 33,1 | 35  |
| 22 | 2779 | 2330 | 23812 | 28,6 | 30  |
| 23 | 2914 | 2445 | 29293 | 22,3 | 45  |

$C_{FB}$ : Enzymkonzentration in der Fermentationsbrühe

$C'_{FB}$ : Enzymkonzentration in der restlichen Fermentationsbrühe nach Adsorption und Verdunstung

8

Tabelle 5

| Versuch | LF | LF' | pH | pH' | verdunstete Fermentations- brühe | aufgegebene Fermentations- brühe |
|---|---|---|---|---|---|---|
| | [mS/cm] | [mS/cm] | [–] | [–] | [%] | [ml] |
| 1 | 1,24 | 1,29 | 6,79 | 6,25 | 77 | 20 |
| 3 | 1,03 | 0,92 | 6,84 | 6,29 | 66 | 15 |
| 4 | 1,11 | 0,91 | 6,78 | 6,25 | 63 | 15 |
| 5 | 1,11 | 0,98 | 6,82 | 6,19 | 61 | 15 |
| 8 | 1,09 | 0,67 | 6,81 | 6,39 | 78 | 15 |
| 10 | 1,06 | 1,13 | 6,81 | 6,37 | 89 | 20 |
| 11 | 1,09 | 1,57 | 6,84 | 6,48 | 69 | 30 |
| 12 | 1,08 | 1,36 | 6,84 | 6,38 | 72 | 30 |
| 13 | 1,05 | 0,97 | 6,83 | 6,29 | 77 | 15 |
| 14 | 1,08 | 0,85 | 6,80 | 6,33 | 65 | 15 |
| 15 | 1,07 | 0,73 | 6,84 | 6,31 | * | 15 |
| 16 | 1,12 | 0,79 | 6,77 | 6,25 | 3 | 15 |
| 17 | 1,10 | 0,78 | 6,77 | 6,29 | 0 | 15 |
| 18 | 1,09 | 1,26 | 6,77 | 6,52 | 79 | 35 |
| 19 | 1.10 | 0,79 | 6,68 | 6,01 | 13 | 7,5 |
| 20 | 1,08 | 0,67 | 6,72 | 6,02 | 59 | 7,5 |
| 21 | 1,06 | 1,33 | 6,68 | 6,31 | 81 | 35 |
| 22 | 1,11 | 1,42 | 6,74 | 6,35 | 65 | 30 |
| 23 | 1,10 | 1,64 | 6,69 | 6,53 | 79 | 45 |

*  Bei dem Versuch 15 wurde die Fermentationsbrühe mit destilliertem Wasser verdünnt (Verdünnungsfaktor 1,44).

pH : pH–Wert

LF : Leitfähigkeit

'   : Bezogen auf die Fermentationsbrühe nach der Adsorption.

Unter Beibehaltung der Versuchsbedingungen von Beispiel 1 konnte lediglich 61 % (Versuch 2) der eingesetzten Enzymmenge desorbiert werden. Dieser Wert konnte durch die Erhöhung der Adsorbensmenge in der zusätzlichen Festbettsäule (Figur 1, Säule 7) von 50 g auf 130 g Fractogel auf eine desorbierte Enzymmenge von bis zu 78 % (Versuch 8) gesteigert werden. Dieser hohe Wert ist vergleichbar mit den in Beispiel 1 erreichten Ausbeuten. Bei Erhöhung der aufgegebenen Fermentationsbrühenmenge wird die Ausbeute geringer, was durch die begrenzte Kapazität des Adsorbens erklärt werden kann.

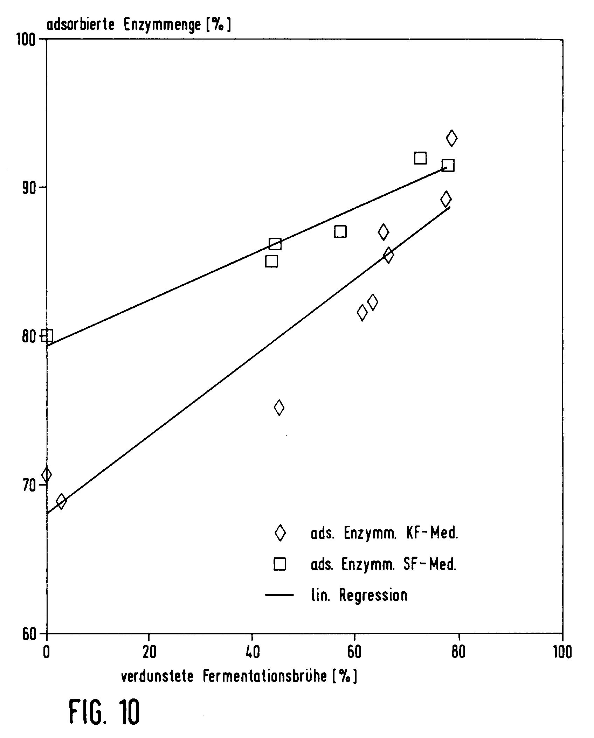

In Figur 10 wird der Einfluß der Verdunstung der Fermentationsbrühe auf den Erfolg des erfindungsgemäßen Verfahrens deutlich: Die adsorbierte Enzymmenge kann durch die Verdunstung um ca. 20 % auf ca.

90 % erhöht werden. Die angegebenen Werte gelten für eine aufgegebene Fermentationsbrühe von 15 ml.

Einen qualitativen Vergleich der Aufreinigungen von Beispiel 1 und Beispiel 2 ermöglicht Figur 8. Trotz unterschiedlicher Fermentationsmedien zeigen beide Eluate einen gleichartigen Verlauf, woraus sich eine gute selektive Trennung der Protease auch bei komplexer Zusammensetzung des Fermentationsmediums erkennen läßt.

Beispiel 3

Aus der in Beispiel 2 angegebenen Fermentationsbrühe wurde die extrazellzulär gebildete $\alpha$-Amylase getrennt. Als Adsorbens wurde der Anionenaustauscher Fractogel EMD (M) TMAE, der Fa. Merck, Darmstadt, mit einer Korngröße von 45 - 90 $\mu$m gewählt. In den Säulen 2 und 7 (Figur 1) wurden jeweils 50 g des Ionenaustauschers gefüllt und mit ca. 200 ml Puffer (Tris - HCl Puffer; pH 8,0; 50 mM) equilibriert. Bei der in Beispiel 1 beschriebenen Vorgehensweise wurde die $\alpha$-Amylase bereits in den ersten Fraktionen der Waschflüssigkeit (destilliertes Wasser) wiedergefunden, während bei der Desorption lediglich Verunreinigungen eluiert wurden Trotzdem führt diese Vorgehensweise zu einer effektiven und qualitativ guten Trennung der $\alpha$-Amylase.

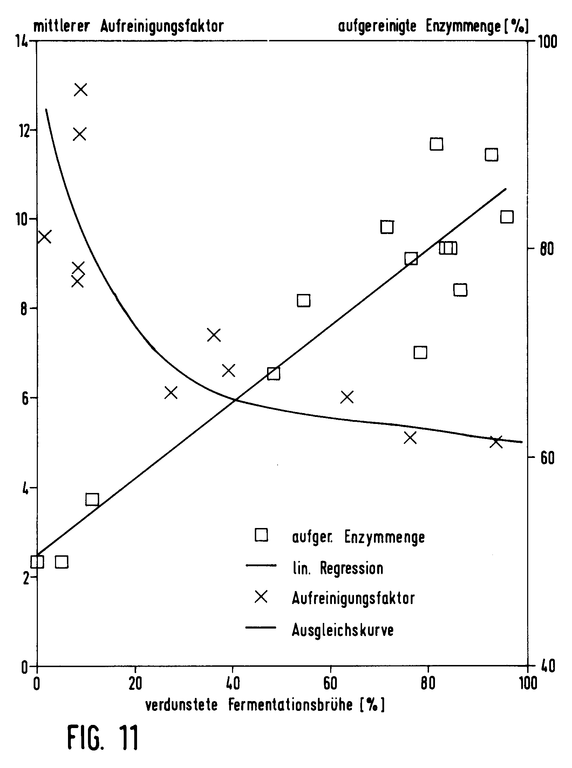

Der positive Einfluß der Verdunstung der Fermentationsbrühe auf die Ausbeute ist in Figur 11 erkennbar, mit zunehmender Verdunstung ist eine Steigerung der aufgereinigten Enzymmenge um 40 % auf maximal 90 % möglich. Die Menge an aufgegebener Fermentationsbrühe beeinflußt die Ausbeute an Enzym nicht, weil die aufgereinigte Enzymmenge nicht durch Kapazität des Austauschers limitiert wird, da das Enzym bereits in den Waschfraktionen wiedergefunden und nicht desorbiert wird.

Eine qualitative Bewertung der Aufreinigung ermöglicht Figur 11: Man kann erkennen, daß bei steigender Menge an Fermentationsbrühe der über die Fraktionen gemittelte Aufreinigungsfaktor sinkt. Dieser Abfall wird durch Verunreinigungen verursacht die durch die begrenzte Kapazität des Austauschers nicht mehr gebunden werden können und somit in die Waschfraktionen gelangen.

Der bei steigender Menge an eingesetzter Fermentationsbrühe maximal erreichte Anreicherungsfaktor liegt bei ca. 2,6.

Beispiel 4

Entsprechend der im Beispiel 3 dargestellten Vorgehensweise wurde eine $\alpha$-Amylase, die von thermophilen Archaebakterien gebildet wurde, mit dem Anionenaustauscher aufgearbeitet. Die $\alpha$-Amylase konnte an den Anionenaustauscher gebunden und mit einer Ausbeute von 95% eluiert werden, obwohl das Fermentationsmedium ca. 3 M NaCl enthält.

Eine stufenweise Elution hat einen positiven Einfluß auf den Aufreinigungsfaktor. Bei Desorption des Enzyms mit einer 1 M NaCl Lösung wird ein über die Eluatfraktionen gemittelter Aufreinigungsfaktor von 1,25 erreicht, während bei Elution mit einer 0,2 M NaCl Lösung ein wesentlich höherer durchschnittlicher Aufreinigungsfaktor von 5 erreicht werden kann.

**Patentansprüche**

1. Verfahren zur Gewinnung von Enzymen aus einer enzymhaltigen Suspension, insbesondere aus unbehandelter Fermentationsbrühe,
**dadurch gekennzeichnet,**
daß man die Suspension mit enzymanlagerungsfähigem Kornmaterial in einem senkrechten Behälter, insbesondere Rohr, zusammenbringt und durch die Masse einen aufsteigenden Inertgasstrom mit einer zur Aufwirbelung ausreichenden Gasgeschwindigkeit unter Durchmischung und Verdunstung hindurchleitet bis zur ausreichenden Anlagerung des Enzyms am Kornmaterial, daß man dann die Suspensionsreste aus der Masse entfernt und das angelagerte Enzym eluiert.

2. Verfahren nach Anspruch 1,
**dadurch gekennzeichnet,**
daß eine wäßrige Suspension behandelt und als Gasstrom ein trockener Stickstoffstrom vorgesehen wird.

3. Verfahren nach Anspruch 1 oder 2,
**dadurch gekennzeichnet,**
daß der Gasstrom und/oder die Masse selbst auf enzymverträgliche Temperaturen aufgeheizt werden.

**4.** Verfahren nach einem der vorangehenden Ansprüche,
**dadurch gekennzeichnet,**
daß als Kornmaterial ein Ionenaustauscher verwendet wird, der auf einen innerhalb des Stabilitätsbereichs des Enzyms liegenden pH-Wert eingestellt ist, der um zumindest eine pH-Einheit von isoelektrischen Punkt des Enzyms abweicht.

**5.** Verfahren nach Anspruch 4,
**dadurch gekennzeichnet,**
daß das Kornmaterial eine Korngröße im Bereich von 50 bis 1000 $\mu$m, insbesondere um 100 $\mu$m aufweist.

**6.** Verfahren nach einem der vorangehenden Ansprüche,
**dadurch gekennzeichnet,**
daß das Hindurchleiten des Gasstroms bis zur - von der Enzymstabilität, dem konzentrationsabhängigen Anlagerungsquotienten (Verhältnis von am Adorbens angelagerter Enzymmenge zur Enzymkonzentration in der Flüssigkeit) sowie von der anlagerungsmindernden Ladungsabschirmung bestimmten - optimalen Enzymbeladung des Kornmaterials erfolgt.

**7.** Verfahren nach einem der vorangehenden Ansprüche,
**dadurch gekennzeichnet,**
daß die Suspensionsreste mittels eines Druckgasstoßes ausgetrieben werden.

**8.** Verfahren nach einem der vorangehenden Ansprüche,
**dadurch gekennzeichnet,**
einen Waschvorgang zwischen Suspensionsrestentfernung und Elution.

**9.** Verfahren nach einem der vorangehenden Ansprüche,
**dadurch gekennzeichnet,**
daß die Suspension portionsweise mit jeweiliger Zwischenverdunstung von Wasser zugegeben wird.

**10.** Verfahren nach einem der vorangehenden Ansprüche,
**dadurch gekennzeichnet,**
daß die aus dem Kornmaterial ablaufende Waschflüssigkeit über eine anschließende Festbettsäule mit enzym-anlagerungsfähigem Kornmaterial, insbesondere Ionenaustauscher, gegeben wird.

**11.** Verfahren nach einem der vorangehenden Ansprüche,
**dadurch gekennzeichnet,**
daß die Elution als Stufenelution erfolgt.

**12.** Vorrichtung zur Durchführung des Verfahrens nach einem der Vorangehenden Ansprüche,
**gekennzeichnet**
durhc einen Vertikalrohrreaktor (2) für die Aufnahme von Enzym-Adsorbermaterial mit Mitteln (1, 4) für die Einspeisung von Flüssigkeit und Mitteln (1, 5) zur unteren Gaseinleitung sowie Mitteln zum Auspressen von RestFlüssigkeit und durch eine Wasch- und Elutionsmittelaufgabe und -verteilung sowie -ableitung ggf. über eine nachfolgende weitere Adsorberkolonne.

FIG. 1

equilibrierte Säule

feuchtes Adsorbens

**FIG. 2A**

Injektion der
Fermentationsbrühe

feuchtes Adsorbens
mit Fermentationsbrühe

**FIG. 2B**

Fermentationsbrühe

Enzymanlagerung

Temperierung

Gas (Stickstoff)

Fermentationsbrühe und Adsorbens

Gas  **FIG. 2C**

Elution des angelagerten Enzyms

Elutionsmittel

Adsorbens mit
angelagerten Enzym

Analytik

**FIG. 2D**

FIG. 3

Adsorption bei 40°C, ca. 90min,
44% verdunstet

Fermentationsbrühe
100%

nicht adsorbierte
Enzymmenge 13,9%

Waschverluste
30,4%

FIG.4

Enzymausbeute
56,4%

Adsorption bei 50°C, ca. 30min,
72% verdunstet

Fermentationsbrühe
100%

nicht adsorbierte
Enzymmenge 8,1%

Waschverluste
7,6%

Verluste durch
Desaktivierung
5,6%

FIG.5

Enzymausbeute 78,7%

EP 0 473 011 A2

Stufenelution mit 1 M NaCl, pH 11

FIG. 6

Stufenelution mit 0,2 m NaCl

FIG. 7

FIG. 8

**FIG. 9**

FIG. 10

FIG. 11